(19) **Europäisches Patentamt / European Patent Office / Office européen des brevets**

(11) **EP 3 228 359 A1**

(12) **EUROPEAN PATENT APPLICATION**

(43) Date of publication:
11.10.2017 Bulletin 2017/41

(21) Application number: 16382150.7

(22) Date of filing: 06.04.2016

(51) Int Cl.:
*A61Q 19/02* (2006.01)  *A61Q 19/08* (2006.01)
*A61K 8/92* (2006.01)  *A61K 8/97* (2017.01)
*A61K 8/04* (2006.01)  *A61K 8/11* (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: **Infinitec Activos, S.L.**
**08170 Montornes del Valles-Barcelona (ES)**

(72) Inventors:
• **MOURELLE MANCINI, Marisabel**
**E-08028 Barcelona (ES)**
• **VASCONCELOS PACHECO, Aimée**
**E-08320 Masnou- Barcelona (ES)**

(74) Representative: **ABG Patentes, S.L.**
**Avenida de Burgos, 16D**
**Edificio Euromor**
**28036 Madrid (ES)**

(54) **CHLORELLA COMPOSITIONS AND USE THEROF FOR REDUCING DARK CIRCLES UNDER THE EYES**

(57) The present invention relates to lipidic compositions comprising *chlorella vulgaris* and to their usefulness in improving dark circles under the eyes. The invention also provides methods for preparing said compositions.

**Figure 6**

**Description**

**FIELD OF THE INVENTION**

The present invention generally refers to the field of cosmetic treatment of dark circles under the eyes, and more particularly relates to lipidic compositions comprising *chlorella vulgaris* and to their usefulness in improving dark circles under the eyes. The present invention also provides methods for preparing said compositions.

**BACKGROUND OF THE INVENTION**

The presence of dark circles under the eyes (DCUE), often referred to as peri-orbital hypermelanosis, is a common cosmetic complaint worldwide. Periorbital hyperpigmentation is defined as bilateral, homogeneous hyperchromic macules and patches primarily involving the thin-skinned lower eyelids and also sometimes extending towards the upper eyelids, eyebrows, malar regions, temporal regions and lateral nasal root. The age of onset is usually after puberty or in early adulthood (16-25 years). It is more pronounced in certain ethnic groups and is also frequently seen in multiple members of the same family. Although not rare in males, females are affected to a greater extent possibly owing to hormonal factors. DCUE are an important concern for many individuals, especially women, in some cases even leading to an impairement on quality of life.

Dark circles under the eyes (DCUE) can be caused by a wide variety of factors, some of which are non-pathological in nature, others of which are pathological in nature. Examples of factors leading to DCUE are lack of sleep; stress; fatigue; dehydration; skin ageing, mainly chronological ageing but also photoaging; skin translucency; excessive facial expressions; skin laxity; slowing blood microcirculation or blood stasis, either temporary, especially at night, which leads to formation of a transitory or permanent vascular congestion or reserve under the eyes and an accumulation of blood pigments in the conjunctive tissue, or pathological; vasodilation; exposure to external factors such as heat, tobacco or UV rays; superficial location of vasculature; eye rubbing, which creates repeated superficial trauma, such as allergy provoked eye rubbing or chronic eye rubbing; hyperpigmentation, such as that resulting from non-inflammatory lesions of the periorbital area e.g. melasma, ephelides, lentigo simplex, junctional nevi, solar lentigines ("liver spots") or nevi, or systemic conditions such as metabolic and endocrine disorders, or that resulting from inflammation, particularly inflammatory conditions that disrupt the deep dermal-epidermal junction; oxidative stress; allergic contact dermatitis; anemia; periorbital edema.

Different cosmetic, therapeutic/surgical and laser treatments are employed to ameliorate the undesirable facial appearance resulting from DCUE. Laser and especially surgical treatments are often invasive for the person receiving the treatment. On the other hand, most cosmetic products designed for treating dark circles are tinted with pigments of various colors to cover over or offset the dark of the circles and reflect incident light. They merely cover the existing dark circles.

The use of green microalgae or their extracts in cosmetic compositions to improve skin appearance is long known in the art. The present invention particularly refers to compositions of the Chlorella algae genus. Chlorella occurs in both fresh water and marine water, and has the highest content of chlorophyll of any known plant. It also contains vitamins, e.g. provitamin A, vitamins B1, B2, B6, niacin, Bi2, biotin, vitamin C, vitamin K, pantothenic acid, folic acid, choline, lipoic acid, inositol, PABA; minerals, e.g. P, K, Mg, S, Fe, Ca, Mn, Cu, Zn and Co; dietary fibre; nucleic acids; and amino acids.

The use of *Chlorella* algae has been reported in the literature. For instance, FR 1 125 342 reported the use of *Chlorella* in cosmetic products such as creams.

A continuous need exists to develop improved cosmetic compositions and methods which better the appearance of the skin, and in the context of the present invention, reduce DCUE in a non-invasive and effective manner.

The compositions and methods of the invention are intended to treat DUCE (i.e. the bilateral, homogeneous, hyperchromic macules or patches in the eye-lid area) without affecting any possible pathological condition that may underlie the appearance of said DUCE. That is, the compositions and methods of the invention treat the aesthetic effect of DUCE but not their underlying pathological causes.

**SUMMARY OF THE INVENTION**

The authors of the present invention have surprisingly found that specific compositions comprising *Chlorella Vulgaris* are able to reduce DCUE to a considerably greater extent than other *Chlorella Vulgaris* compositions.

In particular, in a first aspect, the present invention is directed to a particle comprising:

- a first lipid component, wherein the melting point of the first lipid component is comprised in the range 7°C to 25°C;
- a second lipid component, wherein the melting point of the second lipid component is comprised in the range 26°C to 36°C;

- *ascophyllum nodosum* extract;
- *Chlorella vulgaris*.

The particles of the invention are typically prepared as dispersions and incorporated in cosmetic compositions. Therefore, in further aspects, the present invention relates to dispersions comprising the above mentioned particles, as well as to cosmetic compositions comprising said particles or dispersions and a cosmetically acceptable carrier.

As already mentioned, the cosmetic compositions of the present invention are useful in reducing DCUE without affecting their underlying pathological causes. Thus, another aspect of the present invention refers to a non-therapeutic method of reducing dark circles under the eyes, the method comprising applying to a portion of human skin showing dark circles under the eyes a cosmetic composition as mentioned above.

In a yet further aspect, the present invention relates to a method for preparing the above mentioned dispersions, the method comprising the steps of:

a) mixing the first lipid component, the second lipid component and the *ascophyllum nodosum* extract;
b) adding the *Chlorella vulgaris*;
c) heating the mixture resulting from step b) to 37°C or higher;
d) in parallel to steps a)-c), preparing the dispersion medium and heating it to 37°C or higher;
e) mixing, at 37°C or higher, the dispersion medium resulting from step d) and the mixture resulting from step c).

BRIEF DESCRIPTION OF THE DRAWINGS

Figure 1 shows a particle according to the invention.

Figure 2 depicts the particle size distribution for a dispersion according to the invention.

Figure 3 graphically depicts the release profile of *Chlorella vulgaris* for a formulation according to the present invention.

Figure 4 reflects occlusion results obtained at 6, 24 and 48 h after applying a cosmetic composition according to the invention.

Figure 5 shows IWA angle observed for individuals to which a cosmetic composition according to the invention was applied for 28 days.

Figure 6 depicts DCUE area reduction in individuals to which a cosmetic composition according to the invention was applied for 28 days.

**DETAILED DESCRIPTON OF THE INVENTION**

The present invention is directed to a particle comprising:

- a first lipid component;
- a second lipid component;
- *ascophyllum nodosum* extract;
- *Chlorella vulgaris*.

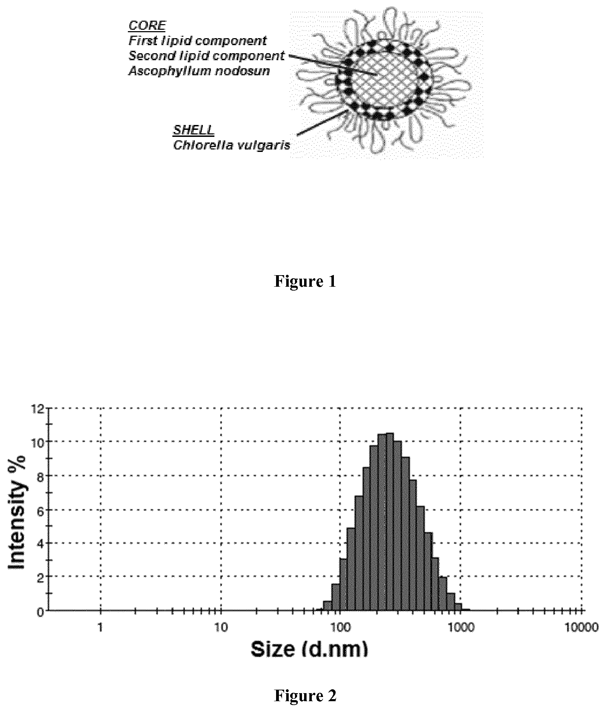

In a preferred embodiment the particle of the invention contains a core and a shell surrounding the core, as depicted in Figure 1. Preferably, the first lipid component, the second lipid component and the *ascophyllum nodosum* extract are comprised in the core.

In a particular embodiment the core consists of the first lipid, the second lipid and the *ascophyllum nodosum* extract. In a preferred embodiment, the *Chlorella vulgaris* is comprised in the shell. In a particular embodiment, the *Chlorella vulgaris* is adsorbed onto the core surface. In another particular embodiment, the core consists of the *Chlorella vulgaris*. In a preferred embodiment the first lipid, the second lipid and the *ascophyllum nodosum* extract are comprised in the core and the *Chlorella vulgaris* is comprised in the shell.

In a particular embodiment, the particle of the invention consists or consists essentially of:

- a first lipid component;
- a second lipid component;
- *ascophyllum nodosum* extract;
- *Chlorella vulgaris*.

Preferred and particular embodiments of the present invention are also applicable to this embodiment.

In the context of the present invention, a lipid component can be formed by a lipid or by a mixture of different lipids.

In a preferred embodiment of the invention, the first lipid component has a melting point of 7°C or higher. More preferably, the melting point of the first lipid component is comprised in the range 7°C to 25°C, i.e. the melting point of the first lipid component is a value or range comprised in the range 7°C to 25°C.

In a preferred embodiment, the second lipid component has a melting point of 36°C or lower. More preferably the melting point of the second lipid component is comprised in the range 26°C to 36°C, i.e. the melting point of the second lipid component is a value or range comprised in the range 26°C to 36°C.

Where a mixture of lipids forms the lipid component, the melting point refers to the melting point of the mixture.

Methods for determining melting points of lipids and lipid mixtures are well-known in the art. In the context of the present invention, melting points refer to the Mettler dropping point (AOCS Method Cc 18-80(09)). In the context of the present invention, room temperature is interpreted to be 25°C.

The lipid or lipids in any lipid component of the present invention are cosmetically acceptable lipids. The term cosmetically acceptable lipid refers to a lipid which is suitable for application to the skin without producing undue toxicity, irritation or allergic response.

Non-limiting examples of cosmetically acceptable lipids which may be included in the lipid mixture forming the lipid component are saturated fats, such as palmitic, stearic, myristic, arachidic or lauric acid and/or triglycerides thereof ; monounsaturated fats, such as palmitoleic or oleic acid; polyunsaturated fats, such as omega-3 fatty acids, e.g. $\alpha$-linolenic acid, or omega-6 fatty acids, e.g. linoleic acid and/or triglycerides thereof.

In a preferred embodiment of the invention, the first lipid component is grape seed oil and/or the second lipid component is selected from cocoa butter and shea butter.

Grape seed oil (also called grapeseed oil or grape oil) is a vegetable oil pressed from the seeds of various varieties of Vitis vinifera grapes, an abundant by-product of winemaking. Grape seed oil typically contains triglycerides derived from the following fatty acids: 69-78% omega-6 (linoleic) acid; 15-20% omega-9 (oleic) acid; 5-11% palmitic acid; 3-6% stearic acid; 0.1-3% omega-3 (linolenic) acid; and 0.5-0.7% omega-7 (palmitoleic) acid.

Cocoa butter is obtained from whole cocoa beans, which are fermented, roasted, and then separated from their hulls. Cocoa butter typically contains triglycerides derived from the following fatty acids: 24-37% stearic acid; 24-30% palmitic acid; 0-4% myristic acid; 1% arachidic acid; 0-1% lauric acid; 29-38% oleic acid; 0-2% palmitoleic acid; 0-4% linoleic acid; 0-1% $\alpha$-linolenic acid.

In a particular embodiment, the melting point of the cocoa butter is comprised in the range 26°C to 36°C. In a preferred embodiment, one or more of polymorphic forms III, IV, V and V of cocoa butter is used.

In the context of the present invention, *Ascophyllum nodosum* refers to the whole or any part of the *Ascophyllum nodosum* seaweed. *Ascophyllum nodosum* extract refers to any extract of the seaweed *Ascophyllum nodosum.* The extract can be obtained by methods known in the art.

In a preferred embodiment, the thallus of *Ascophyllum nodosun* is extracted. In other words, the *Ascophyllum nodosum* extract is a *Ascophyllum nodosum* thallus extract.

In a preferred embodiment, the extraction of *Ascophyllum nodosum* is carried out in organic solvents. In another embodiment, the extraction of *Ascophyllum nodosum* is carried out in lipidic solvents.

In a preferred embodiment, the extract is obtained by extracting *Ascophyllum nodosum* thalli with the first lipid component described in any of the above embodiments, preferably with grape seed oil.

*Ascophyllum nodosum* extracts are furthermore commercially available, for instance from Biogrundl, S.L. (as D (40%) / OVE / T / extract).

Advantageously, in a preferred embodiment, the first lipid component employed for the extraction of *Ascophyllum nodosun* (i.e. the first lipid component containing the extract) is employed in the particles of the invention as the first lipid component and the *Ascophyllum nodosum* extract. Preferably, the first lipid component is grape seed oil.

In the context of the present invention, *Chlorella vulgaris* refers to the whole or any part of the *Chlorella vulgaris* seaweed. Products obtained by extraction (i.e. extracts) of the seaweed are also included in the context of the present invention. A number of Chlorella products are commercially available, for instance from Shaanxi Fuheng(FH) Biotechnology Co.,Ltd as cellwall broken Chlorella powder.

In a preferred embodiment, *Chlorella vulgaris* seaweed (and not an extract thereof) is employed in the particles of the invention. In a particular embodiment, the *Chlorella vulgaris* seaweed used in the particles of the invention has been washed with water. More particularly, washing with water implies that the water is removed and the washed product is collected. This can be achieved by different methods known in the art, e.g. by washing the seaweed with water on a mesh screen permeable to water. Preferably, the washed *Chlorella vulgaris* seaweed is further subjected to spray drying. In a particular embodiment it is further subjected to spray drying to obtain the product in the form of a powder. In a particular embodiment the washed *Chlorella vulgaris* seaweed is spraydried at 160-180°C.

In other embodiments, *Chlorella vulgaris* seaweed is extracted and the extract is employed in the particles of the invention. In a more particular embodiment, the extract is obtained by alkaline hydrolysis of *Chlorella vulgaris* seaweed.

In a particularly preferred embodiment of the invention cocoa butter, grape seed oil and the *ascophyllum nodosum* extract are comprised in the core of the particle, and the *Chlorella vulgaris* is comprised in the shell of the particle.

The particles of the invention may comprise further agents, for instance a film-forming agent, a binder, a bulking agent, a disintegrant, a lubricating agent, a diluent, an osmotic pressure regulator, a pH adjusting agent, a dispersant, an emulsifier, an antiseptic agent, a stabilizer, an antioxidant, a colorant, a thickener, an activity enhancer, a disinfectant, a fragrance, a flavoring agent or an odorizing agent. The particles may also comprise other active ingredients or cosmetic components such as a moisturizer, a skin lightening agent or makeup components (e.g. makeup base, foundation, face powder, blusher, eye shadow, eyebrow pencil, mascara and the like).

The particle embodiments described above may be combined with each other without limitation, so long as not mutually exclusive.

The present invention is also directed to a dispersion comprising particles as described in any of the above embodiments dispersed in a dispersion medium.

The term dispersion is used here as a generic term in its widest sense, and describes the distribution of a discontinuous phase in a continuous phase. The particles of the invention are preferably dispersed in an aqueous medium, preferably in water. In a preferred embodiment the dispersion is an oil-in-water dispersion, wherein the particles of the invention are comprised in, or preferably form, the discontinuous oil phase.

In a particular embodiment, the dispersion of the invention further comprises a surfactant. When the dispersion is prepared the surfactant at least partially adheres to the surface of the dispersed particles. Preferably, the surfactant is an amphiphilic surfactant. Non-limiting examples of suitable amphiphilic surfactants are neutral, positively-charged, or negatively-charged, natural or synthetic phospholipids molecules such as, but not limited to, natural phospholipids including lecithins (e.g. soybean lecithin or egg lecithin), phosphatidylglycerol, phosphatidylinositol, phosphatidylethanolamine, phosphatidic acid, sphingomyelin, diphosphatidylglycerol, phosphatidyls erine, phosphatidylcholine and cardiolipin; synthetic phospholipids including dimyristoylphosphatidylcholine, dimyristoylphosphatidyl ethyl-N-dimethyl propyl ammonium hydroxide, dimyristoylphosphatidylglycerol, distearoylphosphatidylglycerol and dipalmitoylphosphatidylcholine; and hydrogenated or partially hydrogenated lecithins and phospholipids. In a preferred embodiment, the surfactant is a lecithin. In preferred embodiment the lecithin is selected from the group consisting of soy lecithin and sunflower lecithin.

In an embodiment, the *Chlorella vulgaris* in the dispersion is present in a weight percentage of 0.05% to 5% with respect to the total weight of the dispersion. In a preferred embodiment, it is present in a weight percentage of 0.5% to 3% with respect to the total weight of the dispersion. More particularly, it is present in a weight percentage of 2% with respect to the total weight of the dispersion.

In an embodiment, the first lipid component containing the *ascophyllum nodosum* extract is present in a weight percentage of 0.5% to 10% with respect to the total weight of the dispersion. In a preferred embodiment, it is present in a weight percentage of 3% to 7% with respect to the total weight of the dispersion. More particularly, it is present in a weight percentage of 5% with respect to the total weight of the dispersion.

In an embodiment, the second lipid component is present in a weight percentage of 0.5% to 10% with respect to the total weight of the dispersion. In a preferred embodiment, it is present in a weight percentage of 3% to 7% with respect to the total weight of the dispersion. More particularly, it is present in a weight percentage of 5% with respect to the total weight of the dispersion.

Where a surfactant is present in the dispersion, the surfactant is present in a weight percentage of 0.05% to 3% with respect to the total weight of the dispersion. In a preferred embodiment, it is present in a weight percentage of 0.5% to 1.5% with respect to the total weight of the dispersion. More particularly, it is present in a weight percentage of 1% with respect to the total weight of the dispersion.

In more particular embodiments, the weight percentages described above for the different components of the dispersion of the invention can be combined.

In a particular embodiment, cocoa butter, grape seed oil and the *ascophyllum nodosum* extract are comprised in the core of the particle, and the *Chlorella vulgaris* is comprised in the shell of the particle; the particles are dispersed in water; and the dispersion further comprises lecithin. In an even more particular embodiment, the amounts of each component are as follows:

|  | Chlorella ex. | Ascophyllum nodosum ex./ Grape seed oil | Cocoa butter | Lecithin | Water q.s. |
|---|---|---|---|---|---|
| w/w (dispersion) % | 2 | 5 | 5 | 1 | 100 |

The dispersion medium may comprise agents such as a film-forming agent, a binder, a bulking agent, a disintegrant, a lubricating agent, a diluent, an osmotic pressure regulator, a pH adjusting agent, a dispersant, an emulsifier, an antiseptic agent, a stabilizer, an antioxidant, a colorant, a thickener, an activity enhancer, a disinfectant, a fragrance, a flavoring agent or an odorizing agent. The dispersion medium may also comprise other active ingredients or cosmetic components such as a moisturizer, a skin lightening agent or makeup components (e.g. makeup base, foundation, face powder, blusher, eye shadow, eyebrow pencil, mascara and the like).

The present invention is also directed to cosmetic compositions comprising the particles or the dispersion described in any of the above embodiments, and a cosmetically acceptable carrier.

The term cosmetically acceptable carrier refers to a carrier which is suitable for application to the skin without producing undue toxicity, irritation or allergic response.

Examples of suitable carriers are water; dimethyl isosorbide; isopropylmyristate; surfactants of cationic, anionic and nonionic nature; vegetable oils; mineral oils; waxes; gums; synthetic and natural gelling agents; alkanols; glycols; and polyols. Examples of glycols include, but are not limited to, glycerin, propylene glycol, butylene glycol, pentalene glycol, hexylene glycol, polyethylene glycol, polypropylene glycol, diethylene glycol, triethylene glycol, capryl glycol, glycerol, butanediol and hexanetriol, and copolymers or mixtures thereof. Examples of alkanols include, but are not limited to, those having from about 2 carbon atoms to about 12 carbon atoms (e.g., from about 2 carbon atoms to about 4 carbon atoms), such as isopropanol and ethanol. Examples of polyols include, but are not limited to, those having from about 2 carbon atoms to about 15 carbon atoms (e.g., from about 2 carbon atoms to about 10 carbon atoms) such as propylene glycol.

The cosmetic composition of the present invention may be formulated as a lotion, cream, serum, gel, stick, spray, ointment, liquid wash, soap bar, paste, foam, mousse, powder, wipe, patch, strip, hydrogel, aerosol and non-aerosol spray and similar forms suitable for topical application to the skin of the face. In a preferred embodiment, the cosmetic composition is in the form of a cream or lotion. Methods of formulating cosmetic compositions are well known in the art. In a particular embodiment, the dispersion or particles of the invention are simply mixed with a conventional placebo cream.

The cosmetic composition of the present invention may comprise further additives, such as a film-forming agent, a binder, a bulking agent, a disintegrant, a lubricating agent, a diluent, an osmotic pressure regulator, a pH adjusting agent, a dispersant, an emulsifier, an antiseptic agent, a stabilizer, an antioxidant, a colorant, a thickener, an activity enhancer, a disinfectant, a fragrance, a flavoring agent and an odorizing agent. The cosmetic product may also comprise other active ingredients or cosmetic components such as a moisturizer, a skin lightening agent or makeup components (e.g. makeup base, foundation, face powder, blusher, eye shadow, eyebrow pencil, mascara and the like).

The cosmetically acceptable carrier may be employed in combination with other carriers. Amounts of the carrier may range from 80% to 99.5% in weight with respect to the total weight of the cosmetic composition, preferably from 90 to 98%, and in particular it may be 96%.

The dispersion of the invention may be used in an amount of from 0.5% to 10% in weight with respect to the total weight of the cosmetic composition, preferably of from 2 to 5%, and particularly of 4%.

The present invention further relates to a method for preparing a dispersion as described in any of the embodiments mentioned above, the method comprising the steps of:

a) mixing the first lipid component, the second lipid component and the *ascophyllum nodosum* extract;
b) adding the *Chlorella vulgaris*;
c) heating the mixture resulting from step b) to 37°C or higher;
d) in parallel to steps a)-c), preparing the dispersion medium comprising a surfactant and heating it to 37°C or higher;
e) mixing, at 37°C or higher, the dispersion medium resulting from step d) and the mixture resulting from step c).

In an embodiment, the dispersion medium comprising a surfactant (preferably water comprising a surfactant) is added in the amount necessary to achieve the desired concentration of all or any of the other components in the final dispersion.

In a preferred embodiment, the mixing in step e) is carried out by high shear homogenization. In an embodiment, the mixing of step e) involves the dropwise addition of the dispersion medium resulting from step d) to the mixture resulting from step c).

In another embodiment the temperature in steps c), d) and e) is 37-50°C, preferably 40°C.

In an embodiment, the method of preparation further comprises a step f) of cooling the mixture resulting from step e) to room temperature (25°C).

In a preferred embodiment, the first lipid component employed for the extraction of *Ascophyllum nodosum* (i.e. the first lipid component containing the extract) is employed in the preparation method of the invention as the first lipid component and the *Ascophyllum nodosum* extract.

The preparation method of the present invention allows preparing dispersions wherein the particles of the invention have a particle size of 50-1000 nm, and a mean particle size of 200-300 nm, which is advantageously optimal for dermal application.

Furthermore, the polydispersity (PI) values of the particles obtained with the preparation method is around 0.4-0.3. The high homogeneity in the particle size allows the formation of a lipid layer on the skin surface which is also ideal for dermal application.

Additionally, the particles obtained with the preparation method of the invention possess good zeta potentials, particularly in the range -30 to -40 mV, which advantageously makes them stable against particle aggregation (by virtue of electrostatic repulsion amongst the particles), ultimately avoiding loss of cosmetic function.

Additionally, the particles obtained by the method of preparation of the present invention are sustained-release particles (with regard to the release of *Chlorella*).

The invention is also directed to dispersions obtainable by the preparation method of the invention in any of its above described embodiments.

Additionally, the invention is further directed to a method for preparing the particles of the invention, comprising removing the dispersion medium from a dispersion of the invention to isolate the particles therefrom. This may be achieved by methods known in the art, for example by evaporation of the medium for example by freeze-drying. Likewise, the invention is also directed to particles obtainable by this method.

The present inventors have unexpectedly discovered that the particles of the invention, the dispersions of the invention, and the cosmetic compositions of the invention stimulate the expression of Heme Oxygenase type 1 (HO-1) in the skin thereby assisting in the removal of heme catabolites known to be responsible for dark circles under the eye. It has also been surprisingly found that collagen production is stimulated upon application of the particles of the invention, the dispersions of the invention, or the cosmetic compositions of the invention to skin, thereby reducing the appearance of fine lines and wrinkles.

Thus, the present invention is directed to a non-therapeutic (cosmetic) method of reducing dark circles under the eyes, the method comprising applying to a portion of human skin showing dark circles the particles of the invention, the dispersions of the invention, or the cosmetic compositions of the invention. In particular embodiments, the application is to the lower eyelids, upper eyelids, eyebrows, malar regions, temporal regions and/or lateral nasal root.

The invention is also directed to a non-therapeutic (cosmetic) method of preventing dark circles under the eyes, the method comprising applying to a portion of human skin the particles of the invention, the dispersions of the invention, or the cosmetic compositions of the invention. In particular embodiments, the application is to the lower eyelids, upper eyelids, eyebrows, malar regions, temporal regions and/or lateral nasal root.

The term "non-therapeutic" (cosmetic) denotes a use intended mainly for providing a desired aesthetic effect (reduction of DCUE) or preventing an undesired aesthetic effect (appearance of DCUE).

In a particular embodiment of the present invention the non-therapeutic (cosmetic) treatment of the invention is applied to a person who does not suffer from a pathological condition which leads to DCUE, or to a person who has not been diagnosed with a pathological condition which leads to DCUE.

In a particular embodiment, the DCUE are a consequence of skin ageing. More particularly, the skin ageing is chronologic skin ageing. In another particular embodiment the DCUE are a consequence of stress. In another particular embodiment, the DCUE are a consequence of fatigue, particularly of lack of sleep. In a particular embodiment, the DCUE are a consequence of temporary slowing blood microcirculation or blood stasis, particularly in the veins of the lower eyelids, upper eyelids, eyebrows, malar regions, temporal regions and/or lateral nasal root. In a particular embodiment, the DCUE are a consequence of dehydration.

In a preferred embodiment, the non-therapeutic (cosmetic) treatment of the invention additionally reduces skin wrinkles, in particular skin wrinkles in the lower eyelids, upper eyelids, eyebrows, malar regions, temporal regions and/or lateral nasal root.

It is to be understood that the amount of cosmetic composition applied is a cosmetically effective amount, i.e. an amount that can accomplish a reduction of the dark circles.

The cosmetic composition of the invention is applied for a time sufficient to achieve the desired reduction in DCUE. In a particular embodiment, it is applied twice daily for a period of 14 to 28 days.

In a preferred embodiment, the cosmetic composition is applied once or twice per day.

In an embodiment, approximately 100 mg/day of cosmetic composition comprising 2-5% by weight of the particles of the invention are applied.

In an embodiment, the cosmetic composition is applied to a female subject.

In another embodiment, the cosmetic composition is applied to a subject aged 36 or older, more particularly the subject is aged 36 to 57 years old.

The invention also refers to the use of the particles of the invention, the dispersions of the invention, or the cosmetic compositions of the invention, as described in any of the above embodiments, for treating and/or preventing DCUE or a pathological condition leading to DCUE.

The invention also refers to the particles of the invention, the dispersions of the invention, or the cosmetic compositions of the invention, as described in any of the above embodiments, for use as a medicament, more particularly for use in the treatment and/or prohylaxis of DCUE or of a pathological condition leading to DCUE.

The invention also refers to the use of particles of the invention, the dispersions of the invention, or the cosmetic compositions of the invention, as described in any of the above embodiments, in the preparation of a product for preventing or reducing DCUE or a pathological condition leading to DCUE.

The invention also refers to the use of particles of the invention, the dispersions of the invention, or the cosmetic compositions of the invention, as described in any of the above embodiments.

## Methods & examples

*Dispersion production by High Shear Homogenization*

The dispersion of the invention was prepared by the high shear homogenization method. Ascophyllum nodosum/grape seed oil (mixture obtained from Biogrundl, S.L. as D (40%) / OVE / T / extract) and cocoa butter (obtained from Natura Tec - France) were employed as the lipid base. Chlorella (obtained from Shaanxi Fuheng(FH) Biotechnology Co.,Ltd as cellwall broken Chlorella powder) and lipid phase were mixed and heated to 40°C. At the same time, the aqueous phase was heated to the same temperature. The aqueous phase with surfactant lecithin was poured into the lipid-Chlorella phase drop by drop and mixed with ULTRA-TURRAX at a speed of 24,000 rpm. The resulting dispersion was allowed to cool to room temperature and the particles in the dispersion solidified.

The composition of the obtained dispersion (formulation A) in % weight over the total weight of the dispersion is shown below and is compared with a dispersion containing free *Chlorella* (i.e. *Chlorella* not included in the particles of the invention; formulation B).

| Formulation | Chlorella | Ascophyllum nodosum/Vitis vinifera seed oil | Cocoa butter | Lecithin | Water q.s. |
|---|---|---|---|---|---|
| A Dispersion of the invention | 2 | 5 | 5 | 1 | 100 |
| B Comparative dispersion | 2 | - | - | - | 100 |

*Particle size, zeta potential and encapsatlation efficiency analysis*

The size and the zeta potential of the particles of the obtained dispersion were analysed.

Particle size analysis was performed by photon correlation spectroscopy (PCS). PCS yields the mean particle size (Z-ave) and the polydispersity index (PI) that is a measure of the width of the size distribution. The zeta potential ($Z_{pot}$) was determined by the electrophoretic mobility.

Z-ave and $Z_{pot}$ were analyzed using a Zetasizer nano ZS (Malvern Instruments, Malvern, UK).

The encapsulation efficiency (EE) of *Chlorella* in the particles of the invention was determined indirectly by ultrafiltration method using centrifugal filter tubes with a 100 kDa molecular weight cut-off. The amount of encapsulated *Chlorella* was calculated by the difference between the total amount used to prepare the systems and the amount of *Chlorella* that remained in the aqueous phase after isolation of the systems, applying equation 1. Analysis of *Chlorella* was performed by UV-spectrophotometric method at the wavelength of 394 nm.

$$E.\,E = \frac{\text{Total amount of Chlorella} - \text{Free amount of Chlorella}}{\text{Total amount of Chlorella}}$$

*Equation 1*

Results were as follows:

| Formulation | Z-ave (nm) | PI | Zpot (mV) | EE (%) |
|---|---|---|---|---|
| A | 263.1±3.7 | 0.340±0.032 | -35.5±8.1 | 89.2±5.7 |

The mean particle size observed is appropriate for dermal application. The high homogeneity in the size of the particles of the invention obtained facilitates the formation of a lipid layer on the skin surface leading to easy occlusion and *stratum corneum* penetration of the *Chlorella.* Figure 2 depicts the particle size distribution observed.

The analysis of the $Z_{pot}$ is a useful tool to predict the physical storage stability of colloidal systems. The negative $Z_{pot}$ value observed for the particles of the invention was in the range -30 mV/ -40 mV, showing that the particles should possess a good physical stability since particle aggregation is not likely to occur owing to electrostatic repulsion between the particles.

*In vitiro release profile of Chlorella from Lipid capsule formulation determined by dialysis*

Static Franz diffusion cells were used to evaluate the amount of chlorella released from free *Chlorella* dispersion (formulation B) and dispersion containing *Chlorella* as part of the particles of the invention (formulation A). The surface area of pigskin was 2.5 cm$^2$ and the volume of the receptor phase was approximately 18 ml. The temperature of the assay was accurately controlled at 32 °C to mimic human skin. The acceptor medium was composed of PBS buffer (pH 7,4), stirred by magnetic bar at 700 rpm to avoid different concentrations within the acceptor medium and to minimize stagnant layers. 25 $\mu$l of formulation A or B was placed onto the membrane in the donor compartment. Samples of 500 $\mu$l were withdrawn at suitable time intervals over 24 hours using a syringe needle, and the same volumes were replaced with freshly prepared acceptor medium. The samples were analyzed by UV-spectrophotometric method. The experiment was done in triplicate.

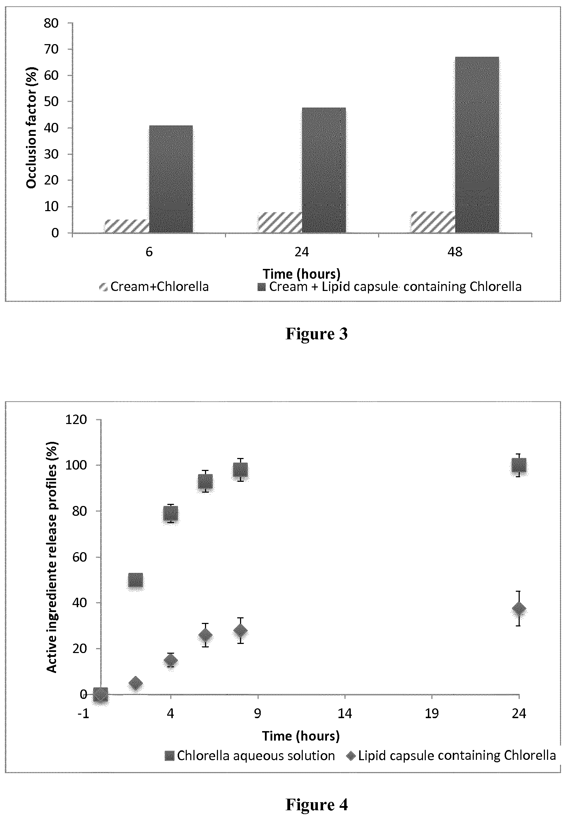

Figure 3 graphically depicts the results obtained. In particular, it can be observed that 100% of Chlorella is rapidly released when active is applied free form (formulation B). However, when the Chlorella is administered as part of the particles of the invention (formulation A) the release is slower. Thus, the administration of chlorella in the particulate form according to the present invention is optimal for achieving an extended release of Chlorella.

*Cream Formulation*

To test in vivo effect creams were prepared by mixing conventional placebo cream with formulation A or B as follows:

- 4% of A (dispersion of the invention) cream
- 4% of B (free *Chlorella;* comparative dispersion) cream
  In both cases the content in *Chlorella* was the same.

*In vitiro Occlusion Test*

This test was performed to compare the *in vitiro* occlusive capacities of cream containing free *Chlorella* (cream of formulation B) and cream containing *Chlorella* as part of the particles of the invention (formulation A). The test was conducted using 10 ml vials, filled with 5 ml distilled water and sealed with cellulose acetate filter with a pore size of 0.45 micrometer. Sample was spread over the filter and stored at 25°C for 6, 24 and 48 h. The occlusion factor F was calculated using the following equation (Equation 2):

$$F = 100 \times [\frac{A - B}{A}]$$

*Equation 2*

where: *A* is the amount of water evaporated through the cellulose acetate membrane without applying a formulation and *B* is the amount of water evaporated through the cellulose acetate membrane after applying a sample of a formulation.

The results obtained are depicted in Figure 4. As can be observed, the occlusion factor for the cream containing *Chlorella* as part of the particles of the invention (formulation A) was substantially higher to that of the cream containing free *Chlorella* (cream of formulation B). The particles of the invention favour the formation of a lipid film on the skin surface and promote penetration of *Chlorella*.

*In vivo efficacy test*

The aim of the study is to assess the efficacy of a cream according to the present invention to reduce the pigmentation/darkening of the bags under the eyes in order to obtain an improved appearance. The study to be carried out will be double blind, randomized for 15 female volunteers with their ages ranging between 36 and 57 years old.

The cosmetic cream according to the present invention (cream of formulation A), cream containing free *Chlorella* (cream of formulation B) or placebo were applied twice daily.

The assessment of skin pigmentation was made through the quantitative data gathered during all the volunteer visits: the baseline and the final visit at day 28. Skin pigmentation assessment was performed through image analysis of cross-polarized face photographs. The details of the machinery, information capture and processing of the data is as follows:

Photographs of the face were taken at 0° and $\pm$ 45° with a Nikon D300 on a specifically designed structure on a capturing bench, the Visio 4D (Eotech, France), in order to ensure that camera height, angle and distance to the volunteer remained constant over the study. The volunteer positioning was also performed on the Visio 4D bench, thanks to a head structure that repositions the volunteer based on their ear and chin position. Homogenous diffuse and cross-polarized lighting was obtained through two Elinchrom StyleRX 600 flashes and linear-polarization gel filters when required. Another linear-polarization filter was added to the camera lens on these occasions.

Image analysis was performed with the FrameScan 2,9,9 sofware (Orio Concept, France) in order to obtain pigmentation images and quantify the color coordinates:

Image analysis was performed to assess both the area occupied by the dark circle, as well as the severity of the dark circle. Severity of the dark circle was analyzed by calculating the $IWA_{Newtone}$.

The $IWA_{Newtone}$ (Individual Whiteness Angle) takes into account, at the same time, the concentration of melanin and hemoglobin.

Thus, light skin means a skin less pigmented and/or a skin with less redness. The higher the $IWA_{Newtone}$, the lighter the skin.

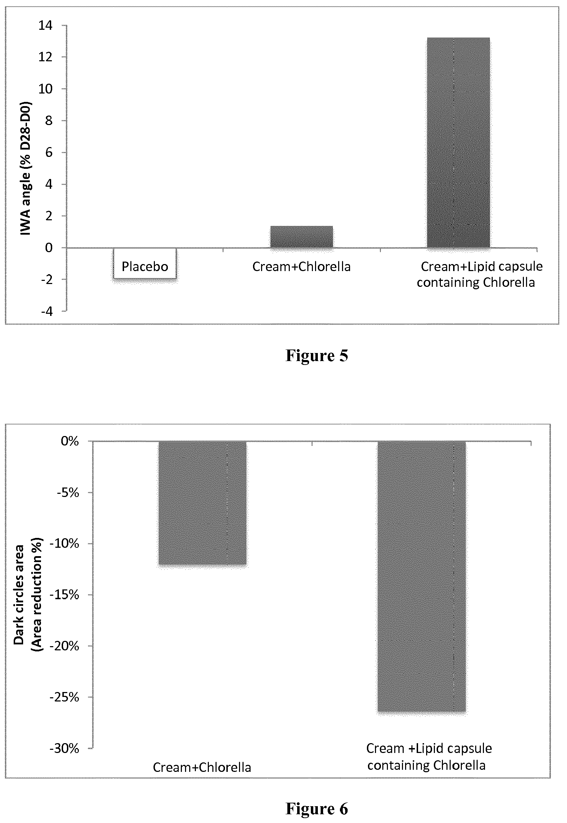

The results observed are depicted in Figures 4 and 5.

The results show that the cream containing *Chlorella* as part of the particles of the invention (formulation A) significantly lightens dark circles as compared to a cream containing free *Chlorella* (cream of formulation B): $IWA_{Newtone}$ is considerably increased (Figure 5) ($p<0.0005$) and the area covered by the dark circles is substantially reduced (Figure 6) for the cosmetic cream of the present invention (cream of formulation A) ($p<0.005$) when compared to the effect of formulation B cream.

**Claims**

1. Particle comprising:

    - a first lipid component, wherein the melting point of the first lipid component is comprised in the range 7°C to 25°C;
    - a second lipid component, wherein the melting point of the second lipid component is comprised in the range 26°C to 36°C;
    - *ascophyllum nodosum* extract;
    - *Chlorella vulgaris*.

2. Particle according to claim 1, wherein the particle contains a core and a shell surrounding the core; wherein the first lipid component, the second lipid component and the *ascophyllum nodosum* extract are comprised in the core; and wherein the *Chlorella vulgaris* is comprised in the shell.

3. Particle according to any one of the preceding claims, wherein the first lipid component is grape seed oil.

4. Particle according to any one of the preceding claims, wherein the second lipid component is cocoa butter.

5. Particle according to any one of the preceding claims, wherein the *ascophyllum nodosum* extract is a grape seed oil *ascophyllum nodosum* extract.

6. Dispersion comprising particles as defined in claims 1-5 dispersed in a dispersion medium comprising a surfactant, preferably in an aqueous dispersion medium comprising a surfactant, more preferably in water comprising a surfactant.

7. Dispersion according to claim 6, wherein the *Chlorella vulgaris* is present in an amount of 0.05-5% by weight with respect to the total weight of the dispersion.

8. Cosmetic composition comprising a dispersion as defined in claims 6 or 7; and a cosmetically acceptable carrier.

9. Cosmetic composition according to claim 8, in the form of a cream.

10. Cosmetic composition according to claim 8 or 9, wherein the dispersion is present in an amount of 2-6% by weight with respect to the total weight of the composition.

11. Method for preparing a dispersion as defined in claims 6 or 7, comprising the steps of:

   a) mixing the first lipid component, the second lipid component and the *ascophyllum nodosum* extract;
   b) adding the *Chlorella vulgaris*;
   c) heating the mixture resulting from step b) to 37°C or higher;
   d) in parallel to steps a)-c), preparing the dispersion medium comprising a surfactant and heating it to 37°C or higher;
   e) mixing, at 37°C or higher, the dispersion medium resulting from step d) and the mixture resulting from step c).

12. Method according to claim 11, wherein the mixing in step e) is carried out by high shear homogenization.

13. Method according to claim 11 or 12, wherein the temperature in steps c), d) and e) is 37-50°C, preferably 40°C.

14. Non-therapeutic method of reducing dark circles under the eyes, the method comprising applying to a portion of human skin showing dark circles a cosmetic composition as defined in any one of claims 8 to 10.

15. The method of claim 14, wherein the cosmetic composition is applied once or twice a day for 28 days.

CORE
First lipid component
Second lipid component
Ascophyllum nodosun

SHELL
Chlorella vulgaris

**Figure 1**

**Figure 2**

**Figure 3**

**Figure 4**

Figure 5

Figure 6

Europäisches
Patentamt

European
Patent Office

Office européen
des brevets

**EUROPEAN SEARCH REPORT**

Application Number

EP 16 38 2150

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | R.H. Müller ,M. Radtke, S.A. Wissing: "Solid lipid nanoparticles (SLN) and nanostructured lipid carriers(NLC) in cosmetic and dermatological preparations", Advanced Drug Delivery Reviews, vol. 54, no. Supplement S1 1 November 2002 (2002-11-01), pages S131-S155, XP002759147, DOI: 10.1016/S0169-409X(02)00118-7 Retrieved from the Internet: URL:http://ac.els-cdn.com/S0169409X0200118 7/1-s2.0-S0169409X02001187-main.pdf?_tid=3 f307708-3931-11e6-860f-00000aab0f27&acdnat =1466679615_7958733b2a260f0df8f63e603b72aa 0a [retrieved on 2016-06-23] * the whole document *<br>----- | 1-15 | INV. A61Q19/02 A61Q19/08 A61K8/92 A61K8/97 A61K8/04 A61K8/11 |
| Y | US 2012/276174 A1 (JOHNSON BENJAMIN [US]) 1 November 2012 (2012-11-01) * the whole document *<br>----- | 1-15 | |
| Y | WO 00/67728 A2 (PHARMASOL GMBH [DE]; MUELLER RAINER HELMUT [DE]; JENNING VOLKHARD [DE]) 16 November 2000 (2000-11-16) * the whole document *<br>----- | 1-15 | TECHNICAL FIELDS SEARCHED (IPC)<br>A61Q A61K |
| Y | WO 2011/116963 A2 (LIPOTEC SA [ES]; VILADOT PETIT JOSEP LLUIS [ES]; DELGADO GONZALEZ RAQU) 29 September 2011 (2011-09-29) * the whole document *<br>-----<br>-/-- | 1-15 | |

The present search report has been drawn up for all claims

| Place of search | Date of completion of the search | Examiner |
|---|---|---|
| Munich | 24 June 2016 | Nopper, Agathe |

EPO FORM 1503 03.82 (P04C01)

page 1 of 3

## EUROPEAN SEARCH REPORT

Application Number

EP 16 38 2150

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE GNPD [Online]<br>MINTEL;<br>May 2014 (2014-05),<br>BeautyCorner: "Vibrant Eye Perfector",<br>XP002759148,<br>Database accession no. 2410563<br>* the whole document *<br>----- | 1-15 | |
| Y | DATABASE GNPD [Online]<br>MINTEL;<br>July 2009 (2009-07),<br>"Regad Délicieux dark Circle Eye Cream",<br>XP002759149,<br>Database accession no. 1137013<br>* the whole document *<br>----- | 1-15 | |
| Y | "Effects of Chlorella Extract on Skin",<br>Personal Care Magazine,<br>November 2007 (2007-11), pages 1-8,<br>XP002759150,<br>Retrieved from the Internet:<br>URL:http://www.osmosisskincare.com/researc h/files/chlorella-extract-on-skin.pdf<br>[retrieved on 2016-06-23]<br>* the whole document *<br>----- | 1-15 | |

TECHNICAL FIELDS
SEARCHED     (IPC)

-/--

The present search report has been drawn up for all claims

| Place of search | Date of completion of the search | Examiner |
|---|---|---|
| Munich | 24 June 2016 | Nopper, Agathe |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

**EUROPEAN SEARCH REPORT**

Application Number

EP 16 38 2150

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Dhanalakshmi. J1, Divakar.R1, Amy Magdalene Paul: "To Study the Potential of Microbial Lipid Based Nanostructured Lipid Carriers for Topical Drug Delivery Applications", International Journal of ChemTech Research International Journal of ChemTech Research, vol. 7, no. 2 2015, 2015, pages 795-799, XP002759151, ISSN: 0974-4290 Retrieved from the Internet: URL:http://sphinxsai.com/2015/ch_vol7_no2_ICONN/5/NB24(795-799).pdf [retrieved on 2016-06-23] ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 24 June 2016 | Nopper, Agathe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 38 2150

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2016

| Patent document cited in search report | | | Publication date | Patent family member(s) | | | Publication date |
|---|---|---|---|---|---|---|---|
| US 2012276174 | A1 | | 01-11-2012 | NONE | | | |
| WO 0067728 | A2 | | 16-11-2000 | AU | 4917900 | A | 21-11-2000 |
| | | | | BR | 0010354 | A | 05-03-2002 |
| | | | | CA | 2369594 | A1 | 16-11-2000 |
| | | | | EP | 1176949 | A2 | 06-02-2002 |
| | | | | JP | 2002544155 | A | 24-12-2002 |
| | | | | MX | PA01011343 | A | 04-06-2002 |
| | | | | TR | 200103188 | T2 | 22-04-2002 |
| | | | | WO | 0067728 | A2 | 16-11-2000 |
| WO 2011116963 | A2 | | 29-09-2011 | EP | 2549977 | A2 | 30-01-2013 |
| | | | | ES | 2384060 | A1 | 29-06-2012 |
| | | | | US | 2013017239 | A1 | 17-01-2013 |
| | | | | WO | 2011116963 | A2 | 29-09-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 1125342